# EUROPEAN PATENT SPECIFICATION

(11) **EP 0 503 002 B1**
(45) Date of publication and mention of the grant of the patent: **11.03.1998**
(21) Application number: 91902031.3
(22) Date of filing: 03.12.1990
(51) Int. Cl.: C12Q 1/70, C12Q 1/68, C07H 21/04, A61K 48/00, G01N 33/53

(54) **ANTISENSE OLIGONUCLEOTIDE INHIBITION OF PAPILLOMAVIRUS**
ANTISENSE OLIGONUKLEOTIDHEMMUNG VON PAPILLOMAVIRUS
INHIBITEURS OLIGONUCLEOTIDIQUES NON CODANTS DU VIRUS DU PAPILLOME

(30) Priority: 04.12.1989 US 445196
(43) Date of publication of application: 16.09.1992
(73) Proprietor: ISIS PHARMACEUTICALS, INC., Carlsbad, CA 92008 (US)
(72) Inventor: CROOKE, Stanley, T., Carlsbad, CA 92009 (US); MIRABELLI, Christopher, K., Encinitas, CA 92024 (US); ECKER, David, J., Carlsbad, CA 92009 (US); COWSERT, Lex, M., Carlsbad, CA 92008 (US)
(74) Representative: Hallybone, Huw George
(86) International application number: US9007067
(87) International publication number: WO9108313

(56) References cited:
- EP-A- 0 301 968
- EP-A- 0 373 352
- WO-A-88/06634
- WO-A-89/05357
- WO-A-91/08132
- US-A- 4 806 463
- US-A- 4 849 331
- US-A- 4 849 332
- US-A- 4 849 334
- US-A- 4 908 306
- US-A- 4 983 728
- EMBO JOURNAL, Vol. 6, issued 1987, BOVER et al. "Differential Promoter Utilization By The Bovine Papillomavirus In Transformed Cells And Productively Infected Wort Tissues", See pages 1027-1035.
- Nature Vol. 299, issued 07 October 1982 (London), CHEN et al., "The Primary Structure and Genetic Organization Of The Bovine Papillomavirus Type 1 Genome", See pages 529-534.

### SEQUENCE LISTING

(1) GENERAL INFORMATION:
   (i) APPLICANT: Crooke, Stanley T., Mirabelli, Christopher K., Ecker, David J., Coswert, Lex M.
   (ii) TITLE OF INVENTION: ANTISENSE OLIGONUCLEOTIDE INHIBITORS OF PAPILLIMAVIRUS
   (iii) NUMBER OF SEQUENCES: 6
   (iv) CORRESPONDENCE ADDRESS:
      (A) ADDRESSEE: Woodcock Washburn Kurtz Mackiewicz & Norris
      (B) STREET: One Liberty Place - 46th Floor
      (C) CITY: Philadelphia
      (D) STATE: PA
      (E) COUNTRY: USA
      (F) ZIP: 19103
   (v) COMPUTER READABLE FORM:
      (A) MEDIUM TYPE: DISKETTE, 3.5 INCH, 1.44 Mb STORAGE
      (B) COMPUTER: IBM PS/2
      (C) OPERATING SYSTEM: PC-DOS
      (D) SOFTWARE: WORDPERFECT 5.0
   (vi) CURRENT APPLICATION DATA:
      (A) APPLICATION NUMBER: PCT/US90/07067
      (B) FILING DATE: December 3, 1990
      (C) CLASSIFICATION:
   (vii) PRIOR APPLICATION DATA:
      (A) APPLICATION NUMBER:
      (B) FILING DATE:
   (viii) ATTORNEY/AGENT INFORMATION:
      (A) NAME: Jane Massey Licata
      (B) REGISTRATION NUMBER: 32,257
      (C) REFERENCE/DOCKET NUMBER: ISIS-0033
   (ix) TELECOMMUNICATION INFORMATION:
      (A) TELEPHONE: (215) 568-3100
      (B) TELEFAX: (215) 568-3439
(2) INFORMATION FOR SEQ ID NO: 1:
   (i) SEQUENCE CHARACTERISTICS:
      (A) LENGTH: 15
      (B) TYPE: nucleic acid
   (xi) SEQUENCE DESCRIPTION: SEQ ID NO: 1:
   GCTTCCATCT TCCTC 15
(2) INFORMATION FOR SEQ ID NO: 2:
   (i) SEQUENCE CHARACTERISTICS:
      (A) LENGTH: 16
      (B) TYPE: nucleic acid
   (xi) SEQUENCE DESCRIPTION: SEQ ID NO: 2:
   GCTTCCATCT TCCTCG 16
(2) INFORMATION FOR SEQ ID NO: 3:
   (i) SEQUENCE CHARACTERISTICS:
      (A) LENGTH: 17
      (B) TYPE: nucleic acid
   (xi) SEQUENCE DESCRIPTION: SEQ ID NO: 3:
   TGCTTCCATC TTCCTCG 17
(2) INFORMATION FOR SEQ ID NO: 4:
   (i) SEQUENCE CHARACTERISTICS:
      (A) LENGTH: 18
      (B) TYPE: nucleic acid
   (xi) SEQUENCE DESCRIPTION: SEQ ID NO: 4:
   TGCTTCCATC TTCCTCGT 18
(2) INFORMATION FOR SEQ ID NO: 5:
   (i) SEQUENCE CHARACTERISTICS:
      (A) LENGTH: 19
      (B) TYPE: nucleic acid
   (xi) SEQUENCE DESCRIPTION: SEQ ID NO: 5:
   TTGCTTCCAT CTTCCTCGT 19
(2) INFORMATION FOR SEQ ID NO: 6:
   (i) SEQUENCE CHARACTERISTICS:
      (A) LENGTH: 20
      (B) TYPE: nucleic acid
   (xi) SEQUENCE DESCRIPTION: SEQ ID NO: 6:
   TTGCTTCCAT CTTCCTCGTC 20

## Description

### FIELD OF THE INVENTION

This invention relates to the inhibition of papillomavirus and the diagnosis and treatment of infections in animals caused by papillomavirus. This invention is also directed to the detection and quantitation of papillomavirus in samples suspected of containing it. Additionally, this invention is directed to oligonucleotides and oligonucleotide analogs which interfere with or modulate the function of messenger RNA from papillomavirus. Such interference can be employed as a means of diagnosis and treatment of papillomavirus infections. It can also form the basis for research reagents and for kits both for research and for diagnosis.

### BACKGROUND OF THE INVENTION

The papillomaviruses (PV) are widespread in nature and are generally associated with benign epithelial and fibroepithelial lesions commonly referred to as warts. They have been detected in and isolated from a variety of higher vertebrates including human, cattle, rabbits, deer and several avian species. Although these viruses are generally associated with benign lesions, a specific subset of the viruses have been associated with lesions that may progress to carcinomas. The implication that these viruses may play a etiologic role in the development of some human cancers follows from numerous studies that have shown the presence of transcriptionally active human papillomavirus (HPV) deoxyribonucleic acids in a high percentage of certain cancerous lesions. Zur Hausen, H. and Schneider, A. 1987. In: The Papovaviridae, vol. 2, edited by N. P. Salzman and P. M. Howley, pp. 245-264. Plenum Press, New York.

In man, human papillomaviruses cause a variety of disease including common warts of the hands and feet, laryngeal warts and genital warts. More than 57 types of HPV have been identified so far. Each HPV type has a preferred anatomical site of infection; each virus can generally be associated with a specific lesion. Genital warts, also referred to as venereal warts and condylomata acuminata, are one of the most serious manifestations of PV infection. As reported by the Center for Disease Control, the sexual mode of transmission of genital warts is well established and the incidence of genital warts is on the increase. The seriousness of genital warts is underlined by the recent discovery that HPV DNA can be found in all grades of cervical intraepithelial neoplasia (CIN I-III) and that a specific subset of HPV types can be found in carcinoma *in situ* of the cervix. Consequently, women with genital warts, containing specific HPV types are now considered at high risk for the development of cervical cancer. Current treatments for genital warts are inadequate.

There is a great, but as yet unfulfilled, desire to provide compositions of matter which can interfere with papillomavirus. It is similarly desired to achieve methods of therapeutics and diagnostics for papillomavirus infections in animals. Additionally, improved kits and research reagents for use in the study of papillomavirus are needed.

Von Knebel Doeberitz & Gissmann (Haematology and Blood Transfusion **31,** 377-379) teach the use of antisense RNA copies of the complete E6 and E7 genes to inhibit their expression. A decrease in cellular growth was observed, but due to technical problems the expression could not be linked directly to this altered phenotype.

US-A-4806463 teaches antisense oligonucleotides for inhibiting HTLV-III replication and expression in cultured human cells. A further aspect of this document is the detection of HTLV-III using these probes.

WO-A-9108312 teaches a method for detecting and quantitating HPV transcripts and/or DNA from the E6 and E7 genes by using oligonucleotide probes to these genes.

EP-A-0301968 teaches a method for using degenerate nucleic acid probes to detect various types of human papillomavirus by hybridisation with the viral DNA, primarily by targeting the E7 gene.

WO-A-8905357 teaches a method for using volume exclusion agents to enhance *in situ* hybridisation rates between short oligonucleotide probes and immobilised target sequences. Thes probes used were prepared against various HPV genes.

### OBJECTS OF THE INVENTION

It is an object of this invention to provide oligonucleotides and oligonucleotide analogs which are capable of hybridising with messenger RNA of papillomavirus to inhibit the function of the messenger RNA.

It is a further object to provide oligonucleotides and analogs which can modulate the functional expression of papillomavirus DNA through antisense interaction with messenger RNA of the virus.

Yet another object of this invention is to provide methods of diagnostics and therapeutics for papillomavirus in animals.

Methods, materials and kits for detecting the presence or absence of papillomavirus in a sample suspected of containing it are further objects of the invention.

Novel oligonucleotides and oligonucleotide analogs are other objects of the invention.

These and other objects will become apparent to persons of ordinary skill in the art from a review of the instant specification and appended claims.

### BRIEF DESCRIPTION OF THE DRAWINGS

Figure 1 is a schematic map of the genetic organization of several PV genomes.

Figure 2 is a partial genetic mapping of a bovine papillomavirus, BPV-1, genome showing open reading frames, ORFs, and messenger RNAs transcribed from the genome.

Figure 3 is a nucleotide sequence of the BPV-1 E2 transactivator gene mRNA showing nucleotides 2443 through 4203.

Figure 4 is a nucleotide sequence of the BPV-1 E2 transactivator gene mRNA showing the domain having nucleotides 2443 through 3080.

Figure 5 is a nucleotide sequence of the 5' common untranslated region of BPV-1 coding for early messenger RNAs showing the domain having nucleotides 89 through 304.

Figure 6 is the nucleotide sequences of antisense oligonucleotides made in accordance with the teachings of the invention and the relative position of the oligonucleotides on the E2 mRNA. The oligonucleotide identifier, sequence and functional role are depicted.

Figure 7 is a graphical depiction of the effects of antisense oligonucleotides made in accordance with the teachings of the invention on E2 expression. Oligonucleotides targeted to the mRNA CAP region (I1751) and the translation codon for the E2 transactivator (I1753) are shown to reduce E2 transactivation at micromolar concentrations.

Figure 8 is a graphical depiction of the dose response of antisense oligonucleotides made in accordance with the teachings of the invention.

These dose response curves show that an antisense oligonucleotide, I1753, which is complementary to the E2 transactivation messenger RNA in the region including the translation initiation codon has an 50% inhibitory concentration (IC₅₀) in the range of 50-100 nM while an oligonucleotide targeted to the CAP region of the same message (I1751) has an IC₅₀ in the range of 500 nM.

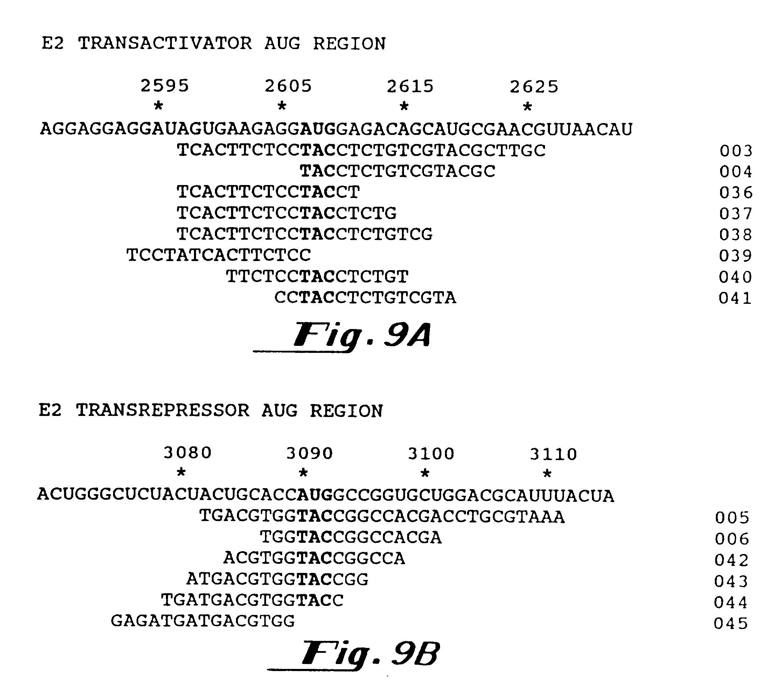

Figure 9 is the nucleotide sequences of antisense oligonucleotides made in accordance with the teachings of the invention targeted to the transactivator and transrepressor regions of the E2 mRNA.

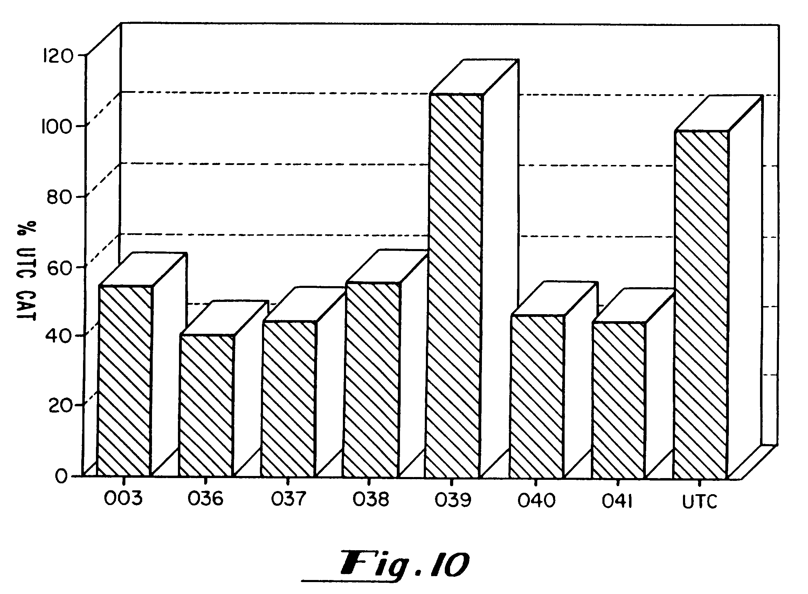

Figure 10 is a graphical depiction of the effects of selected oligonucleotides targeted to the transactivator region of the E2 mRNA. The 15 to 20 mer antisense oligonucleotides made in accordance with the teachings of the invention are shown to inhibit E2 transactivation.

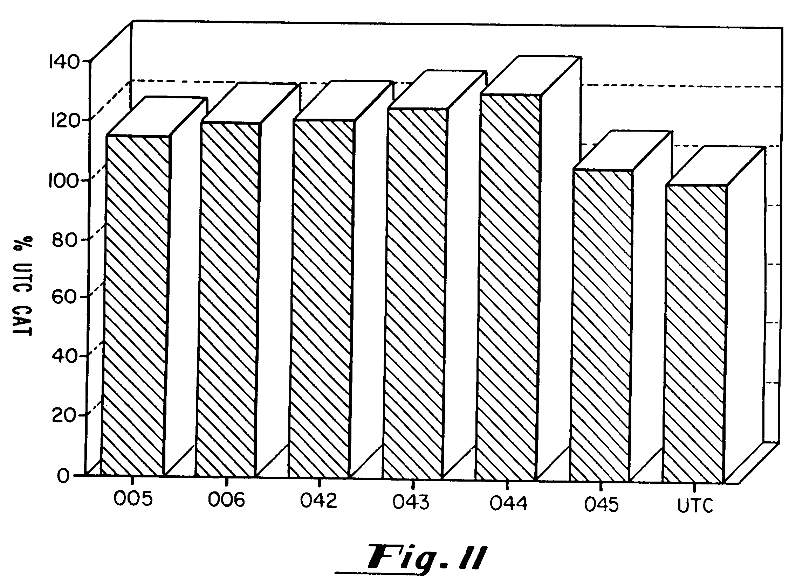

Figure 11 is a graphical depiction of the effects of selected oligonucleotides targeted to the transrepressor region of the E2 mRNA. The 15 to 20 mer antisense oligonucleotides made in accordance with the teachings of the invention are shown to inhibit E2 transrepression.

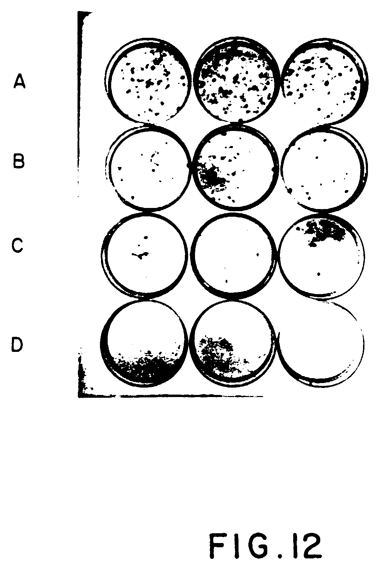

Figure 12 is a photographic depiction of the consequences of reduction of E2 transactivator in *situ* on the biology of BPV-1. Antisense oligonucleotides made in accordance with the teachings of the invention were tested for the ability to inhibit or attenuate BPV-1 transformation of C127 cells. The photograph depicts petri dishes plated with test cells.

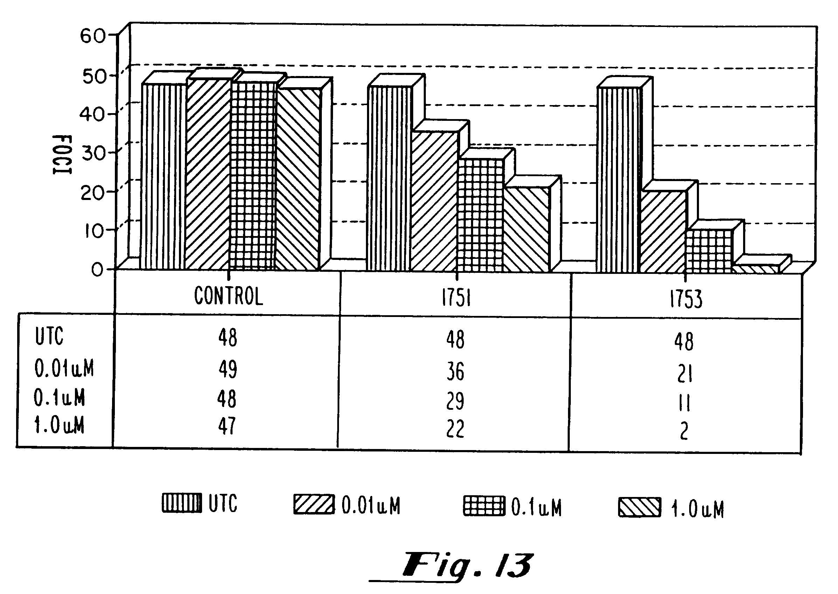

Figure 13 is a graphical depiction of the effects of selected oligonucleotides targeted to the transactivator region of the E2 mRNA. The inhibition of BPV-1 focus formation by antisense oligonucleotides made in accordance with the teachings of the invention is depicted. These dose response curves for I1751 and I1753 show that I1753 had an IC₅₀ in the range of 10 nM while I1751 had an IC₅₀ in the range of 100 nM.

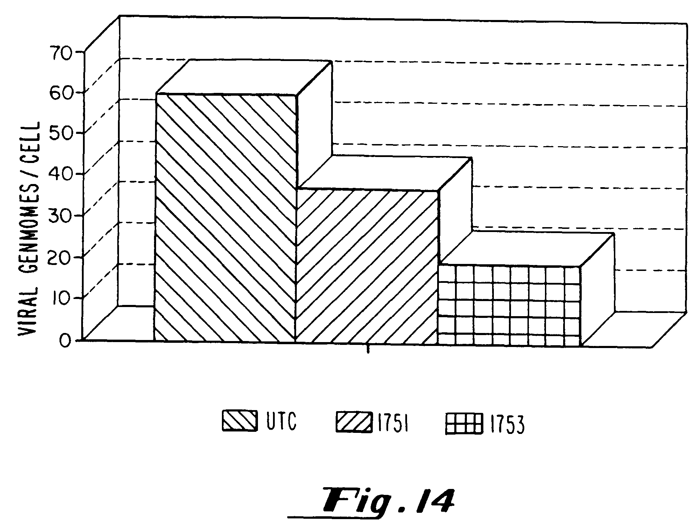

Figure 14 is a graphical depiction of the effects of antisense oligonucleotides made in accordance with the teachings of the present invention on the ability of BPV-1 to replicate its genome. Cells transformed by the virus were treated with I1753 and I1751 and the viral DNA quantitated.

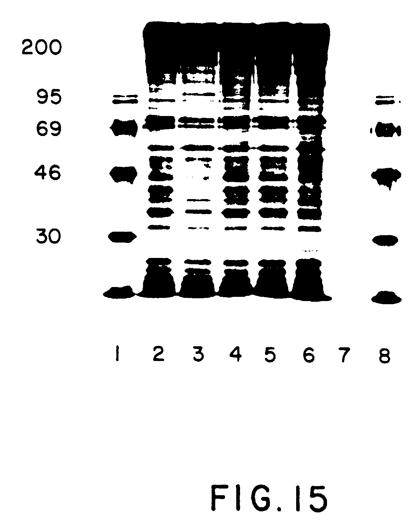

Figure 15 shows the results of immunoprecipitation assays wherein metabolically labelled oligonucleotide-treated cells or untreated controls were immunoprecipitated using a monoclonal antibody.

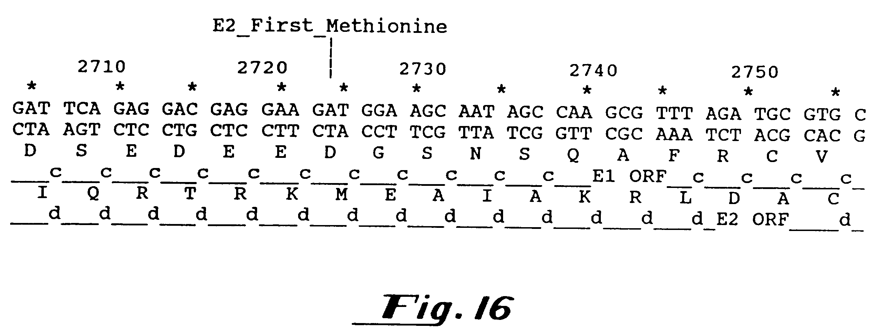

Figure 16 is the nucleotide sequence of HPV-11 in the region of the translation initiation codon of E2.

### SUMMARY OF THE INVENTION

In accordance with preferred embodiments of this invention, oligonucleotides and oligonucleotide analogs are provided which are hybridizable with messenger RNA from papillomavirus. This relationship is commonly denominated as "antisense." The oligonucleotides and oligonucleotide analogs are able to inhibit the function of the RNA, either its translation into protein, its translocation into the cytoplasm, or any other activity necessary to its overall biological function. The failure of the messenger RNA to perform all or part of its function results in failure of the papillomavirus genome to be properly expressed; multiplication fails, correct progeny are not formed in effective numbers, and the deleterious effects of those progeny upon animals infected with the papillomavirus are modulated.

It has now been found to be preferred to target portions of the E2 mRNA of papillomaviruses for antisense attack. Thus, the messenger RNA with which hybridization by the oligonucleotide or oligonucleotide analog is desired, is messenger RNA E2. In accordance with still more preferred embodiments, oligonucleotides and oligonucleotide analogs are provided which comprise nucleotide base sequences designed to be complementary with RNA portions transcribed from the E2 transactivator region or the 5' common untranslated region of the papillomavirus as exemplified, in bovine papillomavirus-1, by nucleotides 2443 through 3080 or 89 through 304.

In the most preferred embodiment, oligonucleotides are provided which are targeted to the mRNA CAP region and the translation codon for the E2 transactivator. These oligonucleotides are designed to hybridize with E2 mRNA encoded by BPV-1 nucleotides 2443 through 4180.

Methods of modulating the expression of papillomavirus have now been discovered comprising contacting messenger RNA from said papillomavirus with an oligonucleotide or oligonucleotide analog hybridizable with a messenger RNA from the papillomavirus, which oligonucleotide or oligonucleotide analog inhibits the function of said messenger RNA when hybridized therewith. Employment of oligonucleotides or oligonucleotide analogs which are designed to hybridize with the E2 mRNAs of papillomavirus are preferred.

Additionally, methods of modulating the effects of a papillomavirus infection in an animal have now been discovered comprising contacting the animal with an oligonucleotide or oligonucleotide analog hybridizable with a messenger RNA from a papillomavirus, that inhibits the function of said messenger RNA when hybridized therewith. Oligonucleotides or oligonucleotide analogs hybridizable with E2 mRNA of papillomavirus are preferred.

Diagnostics for detecting the presence or absence of papillomavirus employing such oligonucleotides or oligonucleotide analogs are also within this invention as are kits for such diagnostic activity and research reagents depending upon such hybridization.

### DETAILED DESCRIPTION OF THE INVENTION

Genital warts are the most frequently diagnosed, viral, sexually transmitted disease. Clinically, they may be categorized into two major groups: condyloma acuminata and flat cervical warts. Condylomas have been shown to contain virus particles and molecular studies have demonstrated that greater than 90% of these lesions contain either HPV-6 or HPV-11 DNA. Gissmann, L., Wolnik, L., Ikenberg, H., Koldovsky, U., Schnurch, H.G. & zur Hausen, H. Proc. Natl. Acad. Sci. USA 80, 560-563 (1983). Condyloma acuminata generally occur on the penis, vulva or in the perianal region. They may spontaneously regress or persist for years and progression to an invasive carcinoma occurs only at a low frequency. Unlike other genital warts, those occurring on the uterine cervix usually exhibit a flat rather than acuminate morphology, and are usually clinically detected by Pap smear. A papillomavirus etiology for cervical dysplasia was suggested by the studies of cytologists in the late 1970s who demonstrated the association on Pap smear of cytologic changes due to HPV infection with those of dysplasia. Other studies showed the presence of viral particles and viral capsid antigen in some of the dysplastic cells of these lesions. This association was important because previous clinical studies had established that cervical dysplasia (also referred to as CIN, or cervical intra-epithelial neoplasia) was a precursor to carcinoma in *situ* which was in turn recognized to be a precursor to invasive squamous epithelial cell carcinoma of the cervix. HPV-types 16 and 18 were cloned out directly from cervical carcinoma. Dürst, M., Gissmann, L., Ikenberg, H. & zur Hausen, H., Proc. Natl. Acad. Sci. USA **80,** 3812-3815 (1983); Boshart, M., Gissmann, L., Ikenberg, H., Kleinheinz, A., Scheurlen, W. & zur Hausen, H., EMBO J. **3,** 1151-1157 (1984). These were subsequently used as hybridization probes to show that greater than 70% of the human cervical carcinomas and the derived cell lines scored positive for the presence of either of these HPV types. Another 20% contain additional HPV-types such as HPV-31, HPV-33, and HPV-35.

Data collected from the National Therapeutic Index showed that in 1984 there were 224,900 first office visits for genital warts and 156,720 first office visits for genital herpes. The incidence of genital warts has steadily increased throughout the 1970s and 1980s, as was recently demonstrated by an epidemiological study in which the mean incidence from 1950 to 1978 reached a peak of 106.5 per 100,000 population. The prevalence of cervical HPV infection in women aged 25 to 55 proved to be 0.8%, but in 22 year old women it was 2.7%. Recent studies on cytologically normal women have demonstrated the incidence of latent infection to be 11%. Thus, there appears to be a latent stage of the disease which suggest an even greater incidence and prevalence.

Active genital warts can be identified in approximately 2.5% of pregnant American women, thus being implicated in 60,000 to 90,000 pregnancies annually. HPV infections are more than twice as prevalent in pregnant women. Each year there are an estimated 1,500 new cases of laryngeal papillomatosis, indicating that the risk of infection from mother to newborn is 1:80 to 1:200.

Laryngeal papillomas are benign epithelial tumors of the larynx. Two PV types, HPV-6 and HPV-11, are most commonly associated with laryngeal papillomas. Clinically, laryngeal papillomas are divided into two groups, juvenile onset and adult onset. In juvenile onset it is thought that the neonate is infected at the time of passage through the birth canal of a mother with a genital PV infection. Disease is usually manifest by age 2 and is characterized by the slow but steady growth of benign papillomas that will ultimately occlude the airway without surgical intervention. These children will typically undergo multiple surgeries with the papillomas always reoccurring. Patients will ultimately succumb to complications of multiple surgery. To date there is no curative treatment for juvenile onset laryngeal papillomatosis and spontaneous regression is rare. Adult onset laryngeal papillomatosis is not as aggressive and will frequently undergo spontaneous remission.

The most common disease associated with papillomavirus infection are benign skin warts. Common warts generally contain HPV types 1, 2, 3, 4 or 10. These warts typically occur on the soles of feet, plantar warts, or on the hands. Common skin warts are most often found in children and young adults. Later in life the incidence of common warts decreases presumably due to immunologic and physiologic changes. Plantar warts can often be debilitating and require surgical removal and they frequently reoccur after surgery. To date there is no reliable treatment for plantar warts. Common warts of the hands are unsightly but rarely become debilitating and are therefore not usually surgically treated.

Epidermodysplasia verruciformis (EV) is a rare genetically transmitted disease which is characterized by disseminated flat warts that appear as small reddish macules. A variety of HPV types have been associated with EV. With time approximately one third of EV patients develop squamous cell carcinoma (SCC) of the skin at multiple sites. In general, SCC occurs on sun exposed areas of the skin. Only a subset of EV associated PV is consistently found in SCC, HPV-5 and HPV-8. Genetic predisposition, immunologic abnormalities, and UV irradiation as well as HPV may all contribute to the development of SCC in these patients.

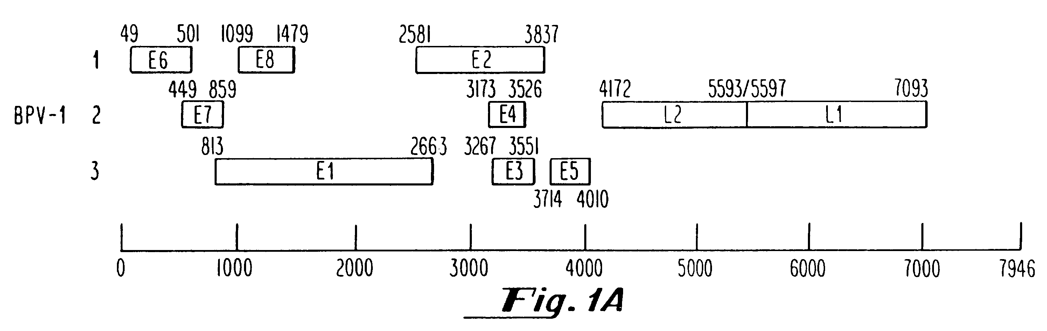

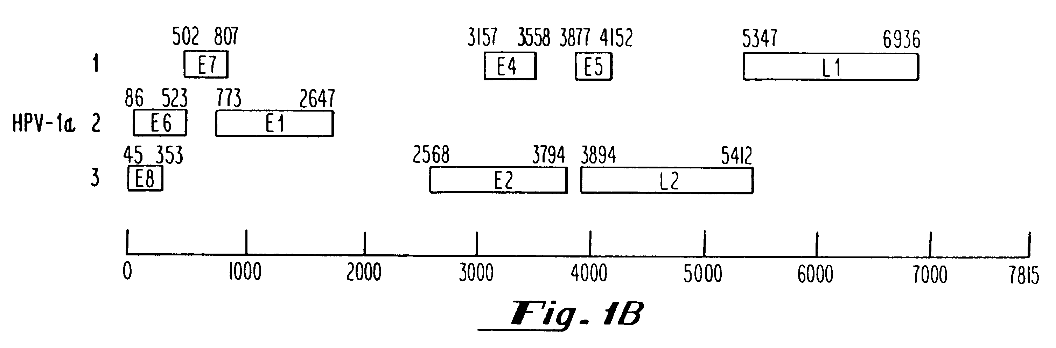

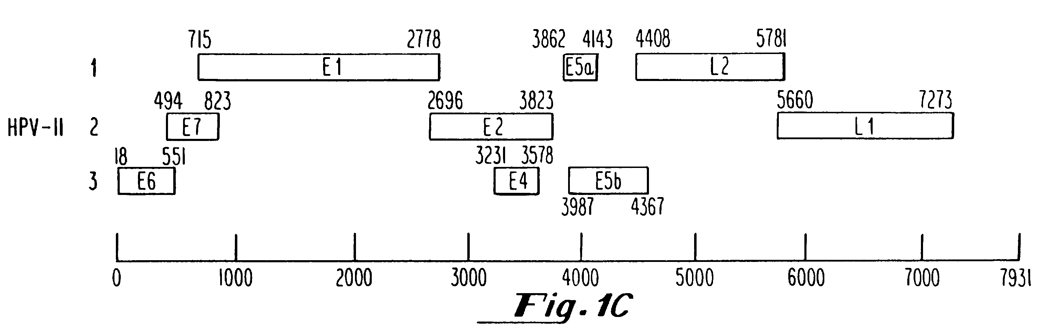

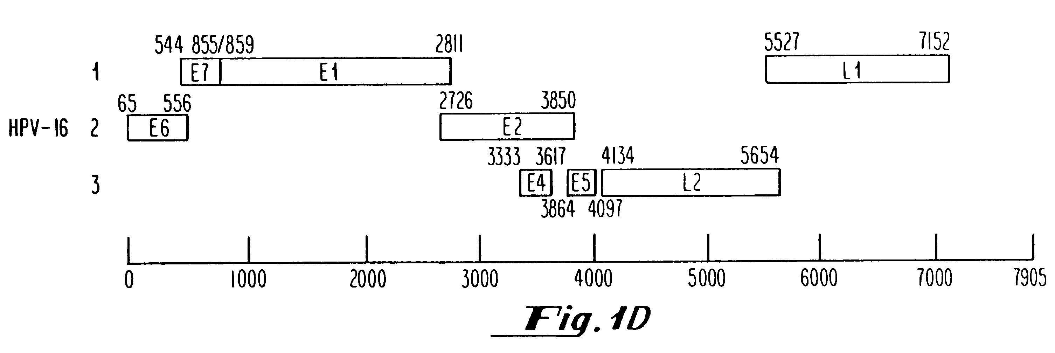

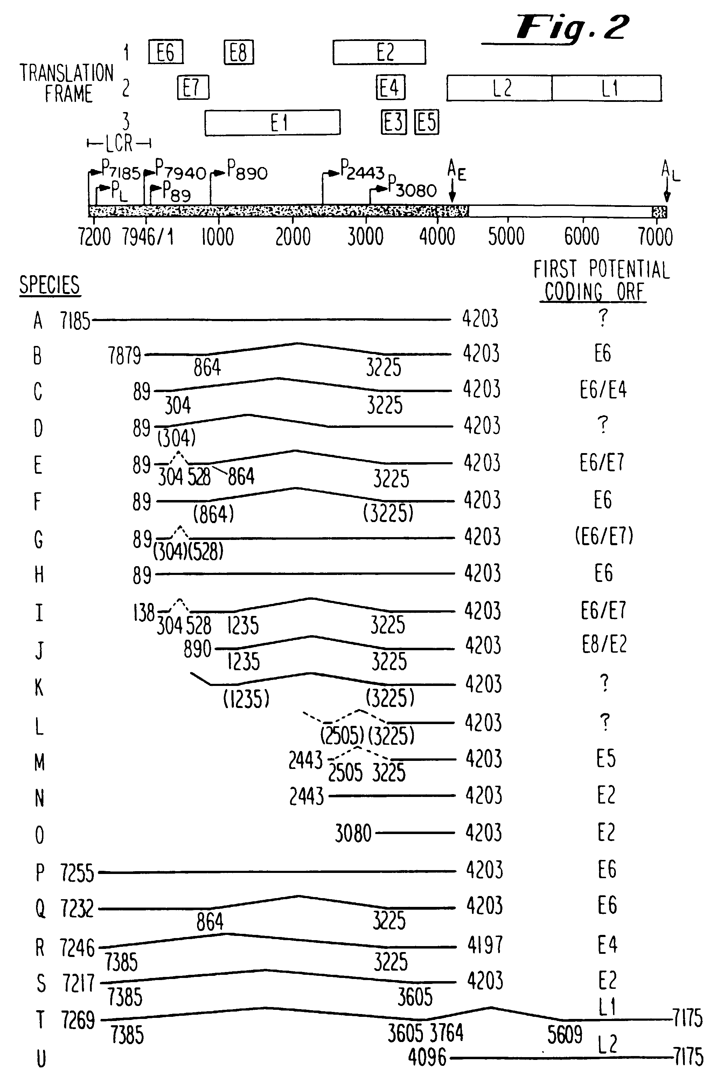

The PV genome consists of a double stranded, covalently closed, circular DNA molecule of approximately 8,000 base pairs. The complete nucleotide sequence and genetic organization of a number of animal and human PVs have been determined including bovine papillomavirus type 1 (BPV-1). Chen, E.Y., Howley, P.M., Levinson, A.D. & Seeburg, P.H., Nature **299,** 529-534 (1982). Schematic maps of several PV genomes are shown in Figure 1. Viral transcription is unidirectional: all viral mRNAs are transcribed from the same strand of viral DNA. Engel, L.W., Heilman, C.A. & Howley, P.M., J. Virol. **47,** 516-528 (1983); Heilman, C.A., Engel, L., Lowy, D.R. & Howley, P.M., Virology **119,** 22-34 (1982). The coding strand contains 10 designated open reading frames (ORFs). The individual ORFs have been classified as either "early" or "late" ORFs based on their position in the PV genome and their pattern of expression in non-productively versus productively infected cells. Figure 2 depicts the relationships of several ORFs for bovine papillomavirus-1.

Because of its ability to transform rodent cells and maintain its genome as an episome in transformed cells, BPV-1 has served as the model papillomavirus in *vitro* studies. As a result, BPV-1 is the best characterized of all the papillomaviruses. The BPV-1 genome is 7946 base pairs in length and has been cloned and sequenced. Chen *et al.*, 1982, supra. DNA sequence analysis of BPV-1 has defined 8 early (E) and 2 late (L) open reading frames (ORFs). Designation of ORFs as early or late was based on their pattern of expression in nonproductively infected transformed cells versus permissively infected cells. Heilman et al., 1982, supra; Baker, C.C. & Howley, P.M. EMBO J. **6,** 1027-1035 (1987).

All ORFs are contained on the same strand of DNA and all mRNAs currently characterized have been shown to be transcribed from the coding strand. Amtmann, E. & Sauer, G., J. Virol. **43,** 59-66 (1982). The functions of the BPV-1 ORFs have been analyzed by recombinant DNA techniques and *in vitro* cell culture systems. Several ORFs have been shown to have multiple functions. The E5 and E6 ORFs have been shown to encode transforming proteins. Yang, Y.C., Okayama, H. & Howley, P.M., Proc. Natl. Acad. Sci. USA **82,** 1030-1034 (1985). The E1 and E7 ORFs are involved in maintenance of high copy number of the BPV-1 genome within the infected cell. Lusky, M. & Botchan, M.R., J. Virol. **53,** 955-965 (1985).

The 3' El ORF encodes a factor required for viral genome replication and maintenance of the viral genome. Lusky, M. & Botchan, M.R., J. Virol. **60,** 729-742 (1986). The 5' E1 ORF encodes a modular of viral DNA replication. Roberts, J.M. & Weintraub, H., Cell **46,** 741-752 (1986). The full length E2 ORF encodes a protein which transactivates viral transcription, (Spalholz, B.A., Yang, Y.C. & Howley, P.M., Cell **42,** 183-191 (1985)) while the 3' E2 ORF encodes a transrepressor of viral transcription. Lambert, P.F., Spalholz, B.A. & Howley, P.M., Cell **50,** 69-78 (1987). No functions for E3, E4, and E8 of BPV-1 have yet been defined.

L1 (Cowsert, L.M., Pilacinski, W.P. & Jenson, A.B., Virology **165,** 613-615 (1988)) and L2 encode capsid proteins.

In accordance with this invention, persons of ordinary skill in the art will understand that messenger RNA identified by the open reading frames of the DNA from which they are transcribed include not only the information from the ORFs of the DNA, but also associated ribonucleotides which form regions known to such persons as the 5' cap region, the 5' untranslated region, and 3' untranslated region. Thus, oligonucleotide and oligonucleotide analogs may be formulated in accordance with this invention which are targeted wholly or in part to these associated ribonucleotides as well as to the informational ribonucleotides.

Within the BPV-1 genome a region of about 1,000 base pairs in length, located between 7,094 and 48 has been identified that has no extensive coding potential. This region is referred to as the long control region (LCR). The LCR contains multiple CIS control elements that are critical for the regulation of viral transcription and viral replication. A summary of functional assignments for the ORFs is set forth in Table 1.

Transcription of the BPV-1 genome is complicated by the presence of multiple promoters and complex and alterative splice patterns. Eighteen different mRNA species have been identified so far by a variety of methods. Amtmann, E. & Sauer, G., J.Virol. **43,** 59-66 (1982); Burnett, S., Moreno-Lopez, J. & Pettersson, U., Nucleic Acids Res. **15,** 8607-8620 (1987); Engel, L.W., Heilman, C.A. & Howley, P.M., J.Virol. **47,** 516-528 (1983); Heilman, C.A., Engel, L., Lowy, D.R. & Howley, P.M., Virology **119,** 22-34 (1982); Baker, C.C. & Howley, P.M., EMBO J. **6,** 1027-1035 (1987); Stenlund, A., Zabielski, J., Ahola, H., Moreno-Lopez, J. & Pettersson, U., J.Mol.Biol. **182,** 541-554 (1985); and Yang, Y.C., Okayama, H. & Howley, P.M., Proc. Natl. Acad. Sci. USA **82,** 1030-1034 (1985).

All early mRNAs appear to use a common polyadenylation signal at nucleotide (nt) 4180 which is positioned down stream of the early ORFs, while late mRNAs use a second polyadenylation signal at nt 7156. Sequence analysis of BPV-1 cDNAs revealed the presence of multiple 5' splice sites (at nt 304, 864, 1234, 2505, 3764, and 7385) and 3' splice sites (at nt 528, 3225, 3605, 5609) resulting in alternative splicing events. The 5' end of most BPV-1 mRNAS map to nt 89 and contain a common region between nt 89 and the first splice donor site at nt 304. The 5' end of other mRNAs map to nts 890, 2443 and 3080.

It is to be expected that differences in the DNA of papillomaviruses from different species and from different types within a species exist. It is presently believed, however, that the similarities among the ORFs of the various PVs as the same might effect the embodiment of the present invention, outweigh the differences. Thus, it is believed, for example, that the E2 regions of the various PVs serve essentially the same function for the respective PVs and that interference with expression of the E2 genetic information will afford similar results in the various species. This is believed to be so even though differences in the nucleotide sequences among the PV species doubtless exist.

Accordingly, nucleotide sequences set forth in the present specification will be understood to be representational for the particular species being described. Homologous or analogous sequences for different species of papillomavirus are specifically contemplated as being within the scope of this invention.

Early genetic experiments showed that deletion or mutation of E2 resulted in loss of BPV focus forming activity on C127 cells suggesting a transforming function for E2. Later studies showed that E2 was a regulator of viral transcription and that loss of transforming ability by mutation of E2 was due to the down regulation of other transforming genes through E2 conditional enhances. The full length E2 ORF encodes the E2 transactivator which stimulates transcription of viral early genes. The E2 transactivator is translated from an unspliced mRNA whose 5'end maps to nt 2443 as shown as species N in Figure 2. The E2 transrepressor is an N-terminally truncated form of the E2 transactivator, generated by initiation of transcription within the E2 ORF by a promoter at nt 3089. Species O, Figure 2. The transactivating (5' portion) and DNA binding domains (3' portion) as well as the palindromic DNA recognition sequence (ACCN6GGT) of E2 have been identified. Both E2 mRNA and protein have been shown to have a very short half life, on the order of less than about 60 minutes. The E2 transregulatory circuit is a general feature among papilloma viruses. E2 transregulator has been documented in every papillomavirus examined to date.

The PV genome is packaged in a naked icosahedral capsid 55 nm in diameter. The viral capsid in nonenveloped and is not glycosylated. Two viral encoded proteins, designated L1 and L2, make up the capsid. L1 is the major capsid protein, constitutes 80% of the protein present in the virion, and has a molecular weight ranging between 50 to 60 kD. The L2 is a minor capsid protein with a theoretical molecular weight of 51 kD but has been shown to migrate at 76 kD. Because PV cannot be produced in *vitro* and very few mature virions are found in productive lesions it has not been possible to do a detailed study of the serology of PV.

The papillomavirus life cycle is complex and poorly understood at this time. To date no in *vitro* system that allows production of mature PV virions has been developed as a result it has not been possible to characterize the life cycle of papillomaviruses. PV have a restricted host range rarely crossing species barriers. In addition PV infect only differentiating epithelium, either mucosal or keratinizing. Regulation of the PV gene expression is thought to be intimately linked to the differentiation program of host epithelial cells. The currently favored model of the PV life cycle is as follows: infectious PV particles penetrate the outer layers of the epithelium via trauma and infect the basal cells of host tissue. There the virus is maintained as a relatively low copy extrachromosomal element. As the epithelial cells begin to undergo differentiation, the PV genome is replicated to high copy number, as the cells begin to undergo the terminal stages of differentiation late genes are expressed and the viral genome is encapsulated.

Papillomavirus has been discovered to be an ideal target for antisense therapy. First, papillomavirus lesions are external, allowing topical approaches to delivery of antisense oligonucleotides and eliminating many of the problems such as rapid clearance, and obtaining clinically active tissue concentrations of oligonucleotides associated with systemic administration of synthetic oligonucleotides. Second, the viral genome is maintained in the infected cell as a separate genetic element. This opens the door to the possibility of curative therapy, as opposed to treatment of symptoms, by attacking replication functions of the virus.

It has been discovered that the E2 ORF on papillomavirus genomes is particularly well-suited for antisense oligonucleotide design. E2 has been shown to be the major transactivator of viral transcription in both BPV-1 and HPV systems. Mutations in the E2 ORF have pleiotropic effects on transformation and extrachromosomal DNA replication. DiMaio, D., J.Virol. **57,** 475-480 (1986); DiMaio, D. & Settleman, J., EMBO J. **7,** 1197-1204 (1988); Groff, D.E. & Lancaster, W.D., Virology **150,** 221-230 (1986); Rabson, M.S., Yee, C., Yang, Y.C. & Howley, P.M., J.Virol. **60,** 626-634 (1986); and Sarver, N., Rabson, M.S., Yang, Y.C., Byrne, J.C. & Howley, P.M., J.Virol. **52,** 377-388 (1984). Subsequently the E2 ORF has been shown to encode a transcriptional transactivator. Spalholz, B.A., Yang, Y.C. & Howley, P.M., Cell **42,** 183-191 (1985). A truncated version of E2 created by initiation of translation at an internal AUG has been shown to be a transrepressor of transcription. Lambert, P.F., Spalholz, B.A. & Howley, P.M., Cell **50,** 69-78 (1987). Both the DNA binding domain, the carboxy terminal 100 amino acids, (McBride, A.A., Schlegel, R. & Howley, P.M., EMBO J. **7,** 533-539 (1988)) and the DNA recognition sequence, ACCN6GGT, (Androphy, E.J., Lowy, D.R. & Schiller, J.T., Nature **325,** 70-73 (1987), and Moskaluk, C. & Bastia, D., Proc.Natl.Acad.Sci.USA **84,** 1215-1218 (1987)) have been identified. The E2 transcriptional regulatory circuit is a general feature among papillomaviruses. E2 transregulation has been documented in each of the other papillomaviruses examined to date. Gius, D., Grossman, S., Bedell, M.A. & Laimins, L.A., J.Virol. **62,** 665-672 (1988); Hirochika, H., Broker, T.R. & Chow, L.T., J.Virol. **61,** 2599-2606 (1987); Chin, M.T., Hirochika, R., Hirochika, H., Broker, T.R. & Chow, L.T., J.Virol. **62,** 2994-3002 (1988); Phelps, W.C. & Howley, P.M., J.Virol. **61,** 1630-1638 (1987); and Thierry, F. & Yaniv, M., EMBO J. **6,** 3391-3397 (1987).

The inventors have determined that the identification of an obligatory viral transcription element that is shared among animal and human papillomaviruses causes E2 to be a prime target for an antisense approach towards papillomavirus research, diagnosis and therapy.

The inventors have determined that the El locus is also promising as a situs for attack upon papillomavirus. Initial mutational analysis of BPV-1 transformation of rodent fibroblast has identified the El as a candidate regulator of viral DNA replication. The 3' El ORF encodes a factor required for viral genome replication and maintenance. Sarver, N., Rabson, M.S., Yang, Y.C., Byrne, J.C. & Howley, P.M., J.Virol. **52,** 377-388 (1984); Lusky, M. & Botchan, M.R., J.Virol. **53,** 955-965 (1985); and Lusky, M. & Botchan, M.R., J.Virol. **60,** 729-742 (1986). Inhibition of expression of El transcription is believed to be likely to inhibit the ability of BPV-1 (and potentially HPV) to replicate their DNA in infected cells.

The inventors have also determined that the E7 site will likely provide a further entré to therapeutics, diagnostics and research into papillomavirus. In BPV-1, E7 has been shown to be involved in regulation of viral DNA replication. Berg, L.J., Singh, K. & Botchan, M., Mol.Cell.Biol. 6, 859-869 (1986). In HPV-16, E7 has been demonstrated to be involved in transformation and immortalization. It is not clear at this time if the E7 of HPVs are involved in replication of viral DNA, however, it is believed that E7 specific antisense oligonucleotides and analogs will inhibit replication of BPV-1 viral DNA.

The E6-E7 region of HPV has been found to be the transforming region. The exact role of this region in the life cycle of the virus is unknown at this time. However, since this region plays a central in the biology of virally induced lesions antisense it has been determined that oligonucleotides targeted to this region are likely to be useful as well.

Recent studies have suggest that antisense oligonucleotides directed towards the 5' regions of mRNAs and preferably the cap region and the start codon are most effective in inhibiting gene expression. One feature of papillomavirus transcription is that many of the mRNAs have a common 5' untranslated region and cap. Thus it has been determined that antisense oligonucleotides directed towards this region have the potential to incapacitate more than one mRNA. The shutting down of multiple viral genes will likely act at a minimum in an additive fashion and possibly synergistically in the eradication of the viral genome from the infected cell.

It will be appreciated that the ORFs of the papillomavirus genome which give rise to the mRNAs which are preferred targets for antisense attack in accordance with the practice of certain preferred embodiments of this invention also encode portions of other mRNAs as well.

The foregoing ORFs are summarized in Table 1.

**TABLE 1**

| Papillomavirus Open Reading Frames and their assigned Functions | |
|---|---|
| ORF | ASSIGNED FUNCTIONS |
| E (BPV-1) | (3'portion) Replication (BPV-1) |
| E2(full length) | Transcriptional transactivation (BPV-1, HPV-6, HPV-16) |
| 3' portion) | Transcriptional repression (BPV-1) |
| E4 | Cytoplasmic phosphoprotein in warts (HPV-1) |
| E5 | Transformation, Stimulation of DNA synthesis (BPV-1) |
| E6 | Transformation (BPV-1) |
| E7 | Plasmid copy number control (BPV-1) |
| L1 | Major capsid protein |
| L2 | Minor capsid protein |

The present invention employs oligonucleotides and oligonucleotide analogs for use in antisense inhibition of the function of messenger RNAs of papillomavirus. In the context of this invention, the term "oligonucleotide" refers to a polynucleotide formed from naturally-occurring bases and cyclofuranosyl groups joined by native phosphodiester bonds. This term effectively refers to naturally-occurring species or synthetic species formed from naturally-occurring subunits or their close homologs.

"Oligonucleotide analog," as that term is used in connection with this invention, refers to moieties which function similarly to oligonucleotides but which have non naturally-occurring portions and which are not closely homologous. Thus, oligonucleotide analogs may have altered sugar moieties or inter-sugar linkages. Exemplary among these are the phosphorothioate and other sulfur containing species which are known for use in the art. In accordance with some preferred embodiments, at least some of the phosphodiester bonds of the oligonucleotide have been substituted with a structure which functions to enhance the ability of the compositions to penetrate into the region of cells where the RNA whose activity is to be modulated is located. It is preferred that such substitutions comprise phosphorothioate bonds, methyl phosphothioate bonds, or short chain alkyl or cycloalkyl structures. In accordance with other preferred embodiments, the phosphodiester bonds are substituted with structures which are, at once, substantially non-ionic and non-chiral. Persons of ordinary skill in the art will be able to select other linkages for use in the practice of the invention.

Oligonucleotide analogs may also include species which include at least some modified base forms. Thus, purines and pyrimidines other than those normally found in nature may be so employed. Similarly, modifications on the cyclofuranose portions of the nucleotide subunits may also occur as long as the essential tenets of this invention are adhered to.

Such analogs are best described as being functionally interchangeable with natural oligonucleotides (or synthesized oligonucleotides along natural lines), but which have one or more differences from natural structure. All such analogs are comprehended by this invention so long as they function effectively to hybridize with messenger RNA of papillomavirus to inhibit the function of that RNA.

The oligonucleotides and oligonucleotide analogs in accordance with this invention preferably comprise from about 3 to about 50 subunits. It is more preferred that such oligonucleotides and analogs comprise from about 8 to about 25 subunits and still more preferred to have from about 12 to about 20 subunits. As will be appreciated, a subunit is a base and sugar combination (or analog) suitably bound to adjacent subunits through phosphodiester or other bonds.

The oligonucleotides and oligonucleotide analogs of this invention are designed to be hybridizable with messenger RNA of papillomavirus. Such hybridization, when accomplished, interferes with the normal function of the messenger RNA to cause a loss of its utility to the virus. The functions of messenger RNA to be interfered with include all vital functions such as translocation of the RNA to the situs for protein translation, actual translation of protein from the RNA, and possibly even independent catalytic activity which may be engaged in by the RNA. The overall effect of such interference with the RNA function is to cause the papillomavirus to lose the benefit of the RNA and, overall, to experience interference with expression of the viral genome. Such interference is generally fatal.

In accordance with the present invention, it is preferred to provide oligonucleotides and oligonucleotide analogs designed to interfere with messenger RNAs determined to be of enhanced metabolic significance to the virus. As explained above, the E2 papillomavirus mRNA is the selected target.

It is also preferred to interfere with RNA coded by nucleotides substantially equivalent to nucleotides 2443 through 3080 or 89 through 304 of bovine papillomavirus-1 genome since these are believed to represent a particularly vulnerable situs for attack as they are believed to code for a plurality of RNAs leading to essential proteins. It will be appreciated that differing papillomaviruses will have somewhat different structures from bovine papillomavirus-1, but that essentially similar areas may be routinely determined.

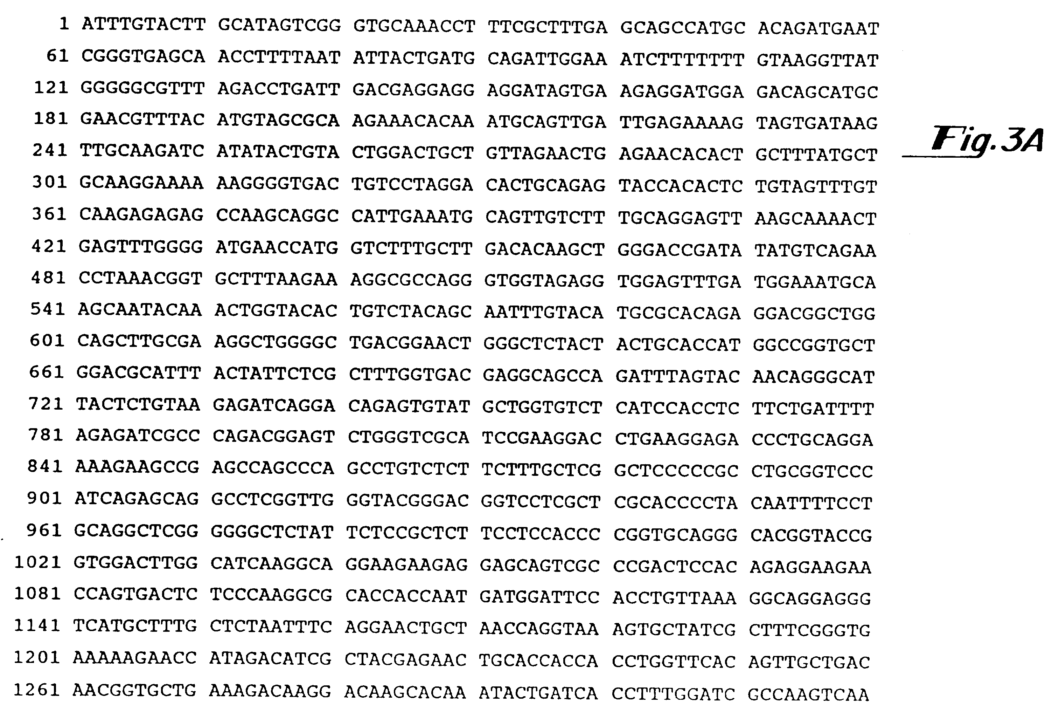

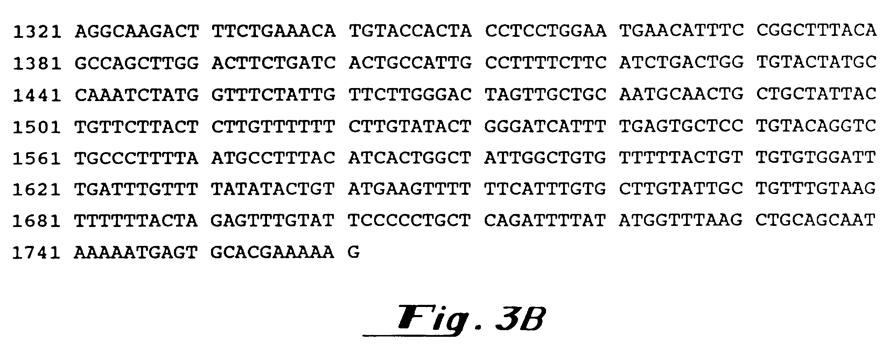

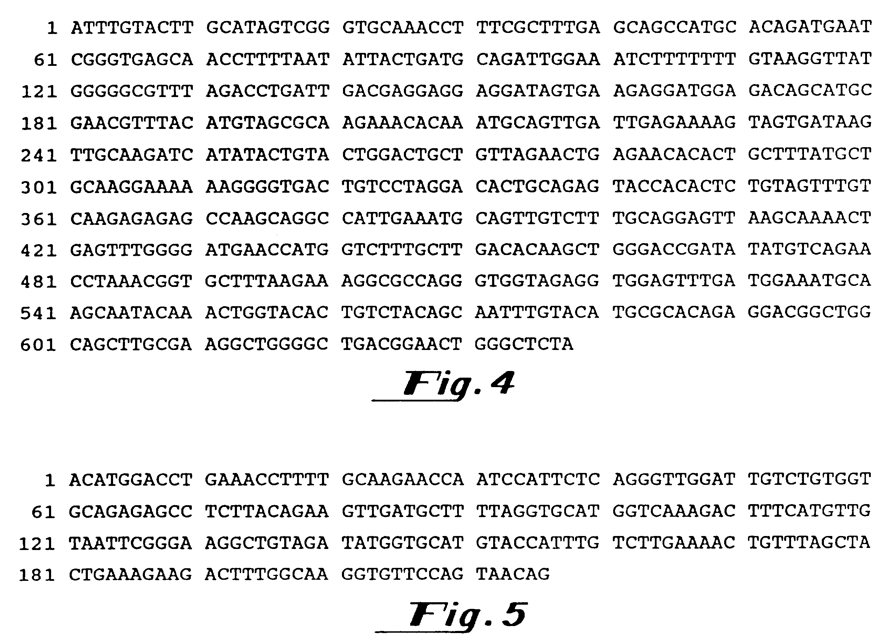

Figures 3, 4, and 5 represent these areas on the bovine papillomavirus-1 genome and any oligonucleotide or oligonucleotide analog designed to interfere with RNA coded by their counterparts in particular papillomaviruses is likely to have especial utility in interfering with operation of those papillomaviruses. Exemplary oligonucleotides targeted at the E2 mRNA of bovine papillomavirus-1 are set forth in Table 2.

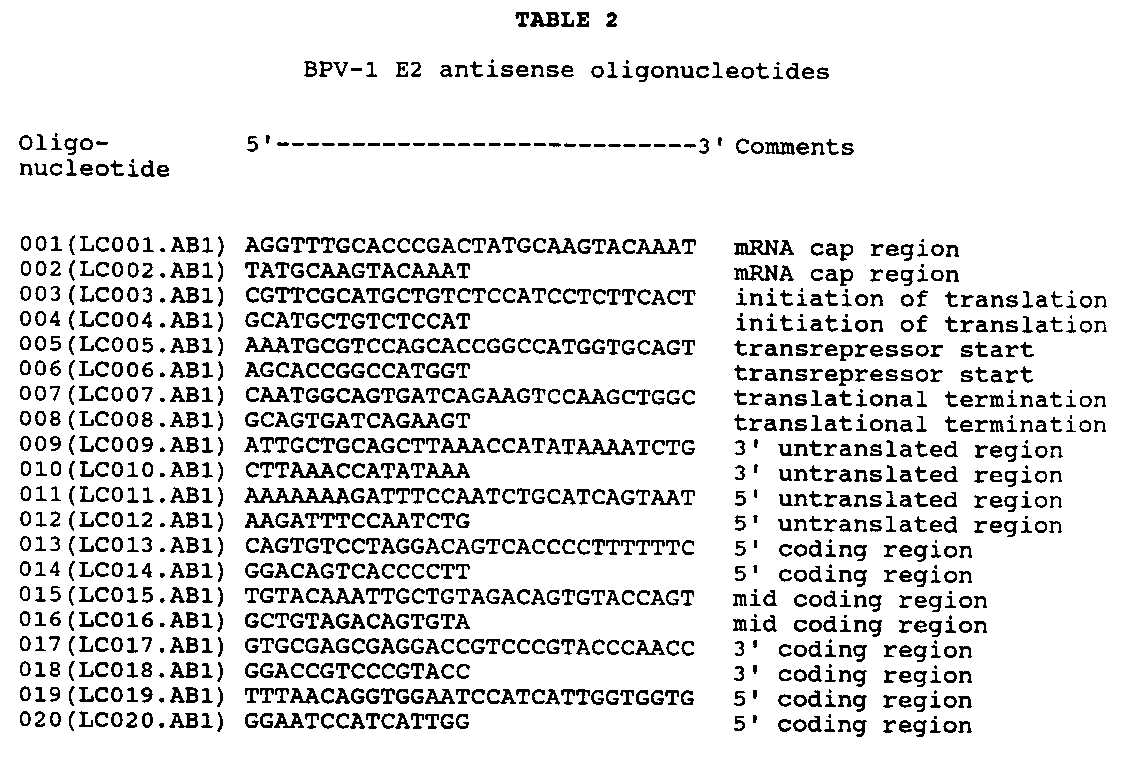

Examplary oligonucleotides targeted to the translation initiation codon of HPV-11 are set forth in Table 3.

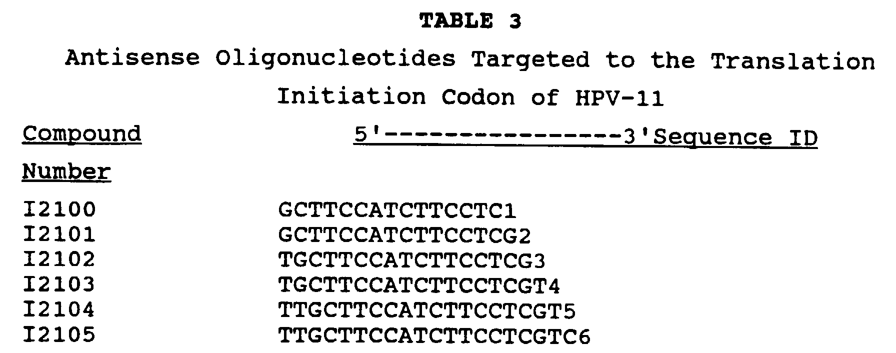

Thus, it is preferred to employ any of the twenty oligonucleotides (or their analogs) set forth above or any of the similar oligonucleotides (or analogs) which persons of ordinary skill in the art can prepare from knowledge of the respective, preferred regions of the E2 ORF of a papillomavirus genome as discussed above.

It is not necessary that the oligonucleotides or analogs be precisely as described in the foregoing table or precisely as required by a slavish interpretation of the mapping of the papillomavirus genome. Rather, the invention permits some digression from strict adherence to the genome structure and it literal "translation" into oligonucleotide. Modifications of such structures may be made so long as the essential hybridizing function of the oligonucleotides and their analogs results.

Similarly, it will be appreciated that species variation among the various papillomaviruses occur. While the various regions, e.g. E2, E1, etc., are very similar from species to species, some differentiation occurs. Alteration in the oligonucleotides and analogs to account for these variations is specifically contemplated by this invention.

The oligonucleotides and analogs used in accordance with this invention may be conveniently and routinely made through the well-known technique of solid phase synthesis. Equipment for such synthesis is sold by several vendors including Applied Biosystems. Any other means for such synthesis may also be employed, however. Useful oligonucleotides may also be conveniently acquired through fermentation techniques, which may be preferred when large amounts of material are desired. The actual synthesis of the oligonucleotides are well within the talents of the routineer.

It is also known to use similar techniques to prepare other oligonucleotide analogs such as the phosphothioates and alkylated derivatives.

A preferred assay to test the ability of E2 specific antisense oligonucleotides to inhibit E2 expression was based on the well documented transactivation properties of E2. Spalholtz et al., J. Virol. 61, 2128-2137 (1987). A reporter plasmid (E2RECAT) was constructed to contain the E2 responsive element, which functions as an E2 dependent enhancer. E2RECAT also contains the SV40 early promoter, an early polyadenylation signal, and the chloramphenicol acetyl transferase gene (CAT). Within the context of this plasmid, CAT expression is dependent upon expression of E2. The dependence of CAT expression on the presence of E2 has been tested by transfection of this plasmid into C127 cells transformed by BPV-1, uninfected C127 cells and C127 cells cotransfected with E2RECAT and an E2 expression vector.

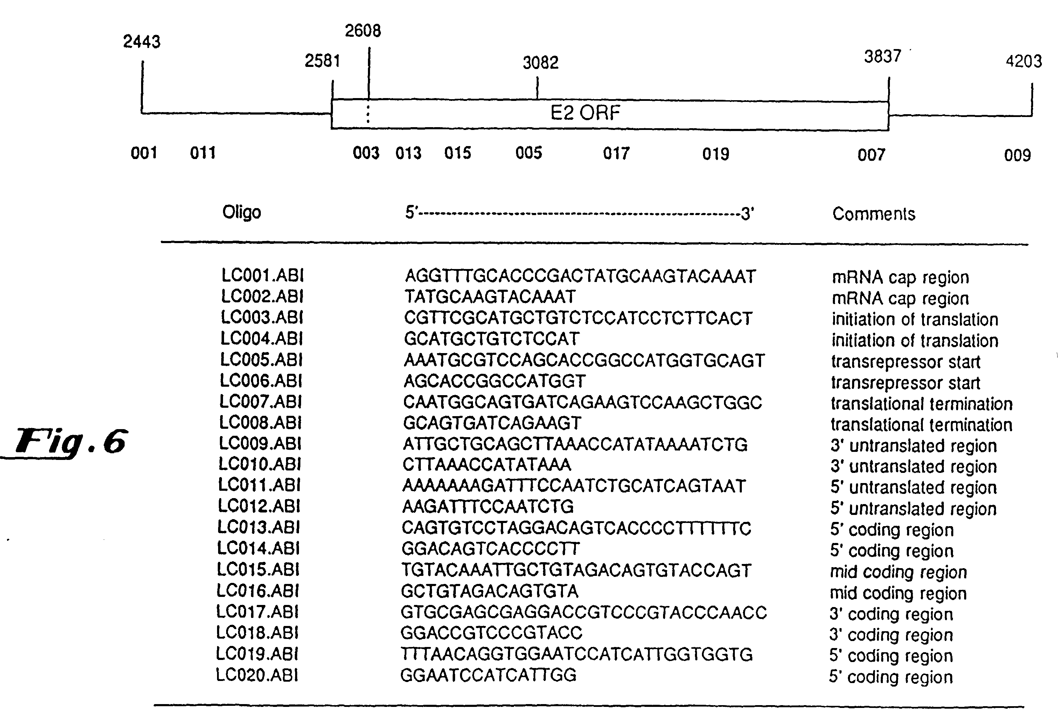

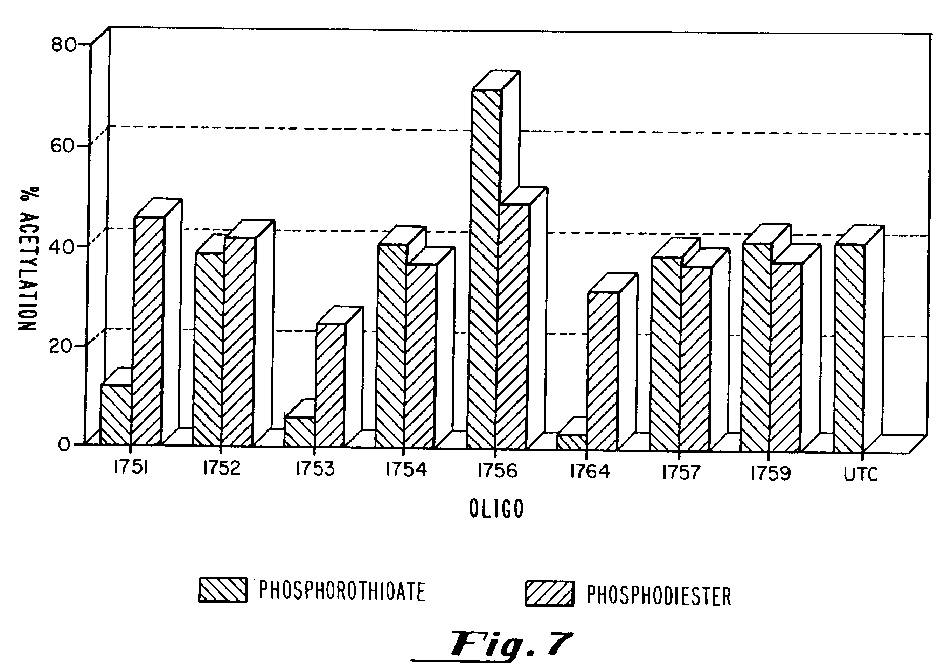

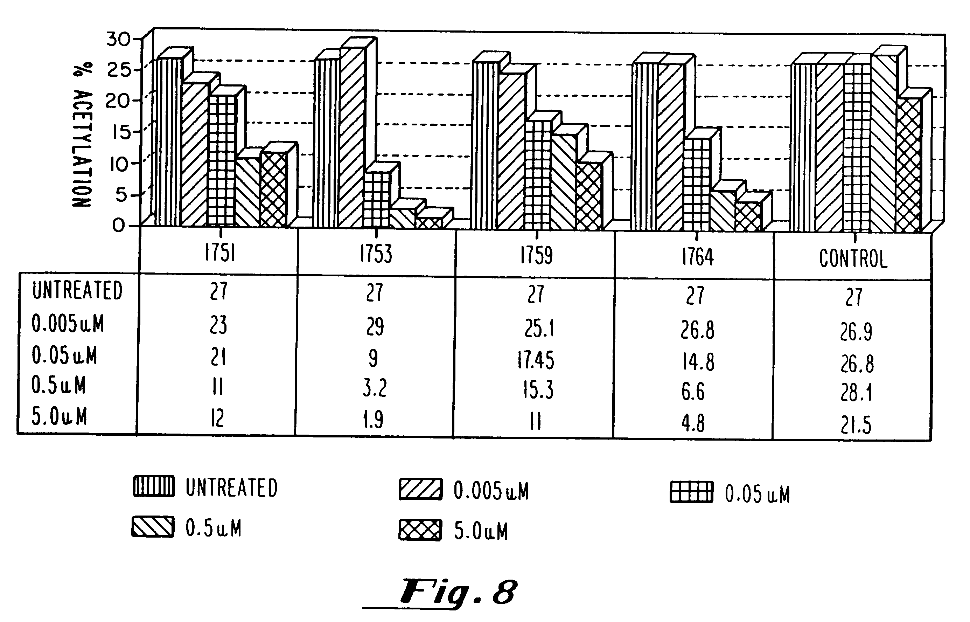

Antisense oligonucleotides were designed to target the major E2 transactivator mRNA (Figure 6). Targets included, but were not limited to, the mRNA CAP region, the translation initiation codon, translation termination codon, and polyadenylation signal. Fifteen or 30 residue oligonucleotides complementary to the various targets were synthesized with a wild type phophosphodiester internucleosidic linkage or a modified phosphorothioate internucleosidic linkage. Oligonucleotides targeted to the mRNA CAP region (I1751) and the translation codon for the E2 transactivator (I1753) were shown to reduce E2 transactivation at 1 micromolar concentrations in preliminary screens (Figure 7). Oligonucleotide I1756 targeted to the translation initiation codon of the E2 transrepressor (Figure 6) was able to give partial relief of transrepression as demonstrated by and increase in CAT activity (Figure 7). Other oligonucleotides of similar length and base composition, but targeted to other areas of the E2 mRNA, as well as other nonsense control, failed to give an antisense effect. In general, oligonucleotides with the phosphorothioate internucleocidic linkage modification were more effective than oligonucleotides of the same sequence containing the natural phosphodiester internucleosidic linkage. This is presumably due to the increased resistance of phosphorothioates to nucleases contained in the serum and within the cell. Dose response curves show that I1753 has an 50 % inhibitory concentration (IC_{**50**}) in the range of 50 to 100 nM while I1751 has an IC_{**50**} ten fold higher in the range of 500 nM (Figure 8). After identification of the translation initiation codon of the E2 transactivator and transrepressor as successful antisense targets an additional set of phosphorothioates were designed to more carefully probe the regions (Figure 9). These data showed that 15 to 20 mer oligonucleotides that covered the appropriate AUG could inhibit either E2 transactivation or transrepression (Figure 10 and 11).

In order to determine the consequences of reduction of E2 transactivator in *situ* on the biology of the BPV-1, antisense oligonucleotides were tested for the ability to inhibit or attenuate BPV-1 transformation of C127 cells (Figure 12). Dose response curves for I1751 and I1753 showed that I1753 had an IC_{**50**} in the range of 10 nM while I1751 had an IC_{**50**} in the range of 100 nM (Figure 13) this 10 fold difference in the IC_{**50**} of these two compounds in this assay is similar to that observed in the inhibition of transactivation assay suggesting that the translation initiation codon is a better target.

To test the effect of E2 targeted antisense oligonucleotides on the ability of BPV-1 to replicate it genome, I-38 cells stably transformed by BPV-1 were treated with I1753 and I1751 and the viral DNA quantitated (Figure 14). After 48 hours of treatment at 1 micromolar concentration the viral DNA copy number on a per cell basis was reduced by factor of approximately 3. During the course of this assay the cells divided between 2 and 3 times. This data suggests that the viral DNA failed to replicate synchronously with the cellular DNA.

In order to test the effect of I1753 on E2 protein synthesis, I-38 cells were metabolically labelled and immunoprecipitated with an E2 specific monoclonal antibody. In cells not exposed to oligonucleotide or cells treated with sense or irrelevant oligonucleotides the 46 kd E2 protein is present (Figure 15). In cells treated with oligonucleotides targeted to the E2, the 46 kd band is lost suggesting that the oligonucleotide is operating by hybridization arrest of translation.

The oligonucleotides and oligonucleotide analogs of this invention can be used in diagnostics, therapeutics and as research reagents and kits. For therapeutic use, the oligonucleotide or oligonucleotide analog is administered to an animal suffering from a papillomavirus infection such as warts of the hands, warts of the feet, warts of the larynx, condylomata acuminata, epidermodysplasia verruciformis, flat cervical warts, cervical intraepithelial neoplasia, or any other infection involving a papillomavirus. It is generally preferred to apply the therapeutic agent in accordance with this invention topically or interlesionally. Other forms of administration, such as transdermally, or intramuscularly may also be useful. Inclusion in suppositories is presently believed to be likely to be highly useful. Use of the oligonucleotides and oligonucleotide analogs of this invention in prophylaxis is also likely to be useful. Such may be accomplished, for example, by providing the medicament as a coating in condoms and the like. Use of pharmacologically acceptable carriers is also preferred for some embodiments.

The present invention is also useful in diagnostics and in research. Since the oligonucleotides and oligonucleotide analogs of this invention hybridize to papillomavirus, sandwich and other assays can easily be constructed to exploit this fact. Provision of means for detecting hybridization of oligonucleotide or analog with papillomavirus present in a sample suspected of containing it can routinely be accomplished. Such provision may include enzyme conjugation, radiolabelling or any other suitable detection systems. Kits for detecting the presence or absence of papillomavirus may also be prepared.

### EXAMPLE 1 Inhibition of Expression of BPV-1 E2 by Antisense Oligonucleotides.

BPV-1 transformed C127 cells are plated in 12 well plates. Twenty four hours prior to transfection with E2RE1 cells are pretreated by addition of antisense oligonucleotides to the growth medium at final concentrations of 5, 15 and 30 mM. The next day cells are transfected with 10 µg of E2RE1CAT by calcium phosphate precipitation. Ten micrograms of E2RE1CAT and 10 µg of carrier DNA (PUC 19) are mixed with 62 µl of 2 M CaCl₂ in a final volume of 250 µl of H₂O, followed by addition of 250 µl of 2X HBSP (1.5 mM Na₂PO₂. 10 mM KCl, 280 mM NaCl, 12 mM glucose and 50 mM HEPES, pH 7.0) and incubated at room temperature for 30 minutes. One hundred microliters of this solution is added to each test well and allowed to incubate for 4 hours at 37°C. After incubation cells are glycerol shocked for 1 minute at room temperature with 15% glycerol in 0.75 mM Na₂PO₂, 5 mM KCl, 140 mM NaCl, 6 mM glucose and 25 mM HEPES, pH 7.0. After shocking, cells are washed 2 times with serum free DMEM and refed with DMEM containing 10% fetal bovine serum and antisense oligonucleotide at the original concentration. Forty eight hours after transfection cells are harvested and assayed for CAT activity.

For determination of CAT activity, cells are washed 2 times with phosphate buffered saline and collected by scraping. Cells are resuspended in 100 ul of 250 mM Tris-HCl, pH 8.0 and disrupted by freeze-thawing 3 times. Twenty four microliters of cell extract is used for each assay. For each assay the following are mixed together in an 1.5 ml Eppendorff tube: 25 µl of cell extract, 5 µl of 4 mM acetyl coenzyme A, 18 µl H₂O and 1 ul ¹⁴C-chloramphenicol, 40-60 mCi/mM and incubated at 37°C for 1 hour. After incubation chloramphenicol (acetylated and nonacetylated forms) are extracted with ethyl acetate and evaporated to dryness. Samples are resuspended in 25 µl of ethyl acetate and spotted onto a TLC plate and chromatograph in chloroform:methanol (19:1). TLC are analyzed by autoradiography. Spots corresponding to acetylated and nonacetylated ¹⁴C-chloramphenicol are excised from the TLC plate and counted by liquid scintillation for quantitation of CAT activity. Antisense oligonucleotides that depress CAT activity in a dose dependent fashion are considered positives.

### EXAMPLE 2 Inhibition of HPV E2 Expression by Antisense Oligonucleotides.

The assay for inhibition of HPV E2 by antisense oligonucleotides is essentially the same as that for BPV-1 E2. For HPV assays appropriate HPVs are co-transfected into either CV-1 or A431 cells with PSV2NEO cells using the calcium phosphate method described above. Cells which take up DNA are selected for by culturing in media containing the antibiotic G418. G418 resistant cells are then analyzed for HPV DNA and RNA. Cells expressing E2 are used as target cells for antisense studies. For each antisense oligonucleotide cells are pretreated as above followed by transfection with E2RE1CAT and analysis of CAT activity as above. Antisense oligonucleotides are considered to have a positive effect if they can depress CAT activity in a dose dependent fashion.

### EXAMPLE 6 Design and Synthesis of Oligonucleotides Complementary to E2 mRNA

Antisense oligonucleotides were designed to be complementary to various regions of the E2 mRNA as defined by the published nucleotide sequence of BPV-1 (Chen, E. Y., Howley, P. M., Levinson, A. D., and Seeburg, P. H. 1982. The primary structure and genetic organization of the bovine papillomavirus type 1 genome. Nature **299**:529-534) and cDNA structure of the major E2 transactivator mRNA (Yang, Y. C., Okayama, H., and Howley, P. M. 1985. Bovine papillomavirus contains multiple transforming genes. Proc. Natl. Acad. Sci. USA **82**:1030-1034). Antisense oligonucleotides targeted to the translation initiation codon of HPV-11 E2 were based on the published sequence of HPV-11 (Dartmann, K., Schwarz, E., Gissamnn, L., and zur Hausen. 1986. Virology **151**:124-130). Solid-phase oligodeoxyribonucleotide syntheses were performed using an Applied Biosystems 380B automated DNA synthesizer. For the phosphorothioate oligonucleotides, sulfurization was performed after each coupling using 0.2 M 3H-1,2-Benzodithiol-3-one-1,1-dioxide dissolved in acetonitrile as described by Iyer et al. (Iyer, R. P., Phillips, L. R., Egan, W., Regan, J. and Beaucage, S. L. 1990. The Automated Synthesis of Sulfur-Containing Oligodeoxyribonucleotides Using ³H-1,2-Bensodithiol-3-one 1,1-Dioxide as a Sulfur-Transfer Reagent. J. Org. Chem. **55**:4693-4699). To insure complete thioation, the growing oligonucleotide was capped after each sulfurization step. After cleavage from the synthesis matrix, deprotection and detriylation oligonucleotides were ethanol precipitated twice out NaCl and suspended in water. The concentration of oligonucleotide was determined by optical density at 260 nm. For use in cell culture assays oligonucleotides were routinely diluted to 100 micromolar stocks and stored at-80°C until use. The purity, integrity, and quantity of the oligonucleotide preparations were determined by electrophoresis on 20% acrylamide 7 M urea gels (40 cm x 20 cm x 0.75 mm) prepared as described by Maniatis et al. (Maniatis, T., Fritsch, E. F. and Sambrook, J. *Molecular Cloning: A Laboratory Manual:* Cold Spring Harbor Laboratory: New York, 1982). Electrophoresed oligonucleotides were visualized within the gel by staining with "Stains-all" , 1-ethyl-2[3-(1-ethylnapthol[1,2-d]thiazolin-2-ylidene)-2-Methyl-Propenyl[napthol[1,2d]thiazolium bromide purchased from Sigma, E-9379, (Dahlberg, A. E., Digman, C. W. and Peacock, A. C. 1969. J. Mol. Biol. **41**:39).

### EXAMPLE 7 Molecular Constructs

The E2 chloramphenicol acetyl transferase (CAT) reporter plasmid used in this study has been previously described (Spalholz, B. A., Byrne, J. C. and Howley, P. M. 1988. Evidence for Cooperativity between E2 Binding Sites in E2 trans-regulation of Bovine Papillomavirus Type 1. J. Virol. **62**:3143-3150). Briefly, the E2 responsive element, E2RE1, (nt 7611-7806) of BPV-1 was reconstructed using oligonucleotides and cloned into pSV2CAT that had been deleted of the SV40 enhancer, Sphl fragment. Expression of CAT from this plasmid has been shown to be dependent upon full length E2. Plasmid C59 contain an E2 cDNA expressed from the simian virus 40 promoter and enhancer and has been described in detail elsewhere (Yang, Y.-C., Okayama, H. and Howley, P. M. 1985. Bovine papillomavirus contains multiple transforming genes. Proc. Natl. Acad. Sci. USA **82**:1030-1034). Two HPV-11 full length E2 expression constructs were made. IPV115 contains the XmnI fragment of HPV-11 (nt 2665-4988) cloned into the SmaI site of pMSG purchased from Pharmacia (catalog number 27-4506), IPV118 contains the same HPV-11 XmnI fragment cloned into the SmaI site of pSVL (Pharmacia, catalog number 27-4509).

### EXAMPLE 8 Cell Lines

Mouse C127 cells (Dvoretzky, I. Schober, R., and Lowy, D. 1980. Focus Assay in Mouse Cells for Bovine Papillomavirus typ 1. Virology **103**:369-375) were grown in Dulbecco's Modified Eagle's medium supplemented with 10% fetal bovine serum, penicillin (100U/ml), streptomycin (100ug/ml), and L-glutamine (4 mM). I-38 cell line was derived from a single focus of C127 cells transformed by purified BPV-1 (Cowsert, L. M., Lake, P., and Jenson, A. B. 1987. Topographical and conformational Epitopes of Bovine Papillomavirus type 1 Defined by Monoclonal Antibodies. JNCI **79**:1053-1057).

### EXAMPLE 9 Oligonucleotide Inhibition of E2 Dependent Transactivation Assays

To test an oligonucleotide's ability to inhibit E2 transactivation or transrepression, I-38 cells were plated at 1 x 10⁴ cells per cm² in 60 mm petri dishes 24 hours before transfection. Sixteen hours prior to transfection media was aspirated and replaced with media containing oligonucleotide at the appropriate concentration. One hour prior to transfection media was aspirated and replaced with fresh media without oligonucleotide. Cells were transfected by the calcium phosphate precipitation method as described by Graham et al. 1973 (Graham, F. L. and van der Eb, A. J. 1973. A New Technique for the Assay of Infectivity of Human Adenovirus 5 DNA. Virology **52**:456-461) with a total of 20 micrograms of DNA in one milliliter of precipitate. Each 60 mm dish received 200 microliters of precipitate containing 4 mictograms of DNA. Four hours after the addition of precipitated DNA the supernatant was aspirated and the cells treated with 15% glycerol (Frost, E. and Williams, J. 1978. Mapping Temperature-Sensitive and host-range mutation of Adenovirus type 5 by Marker Rescue. virology **91**:39-50). After washing cells were refed with media containing oligonucleotide at the original concentration and incubated for 48 hours.

While a number of specific embodiments have been set forth, the present invention is to be limited only in accordance with the following claims.

## Claims

1. An oligonucleotide or oligonucleotide analogue comprising from 8 to 50 bases, hybridisable with a transactivator portion, transrepressor portion, cap region or 5' untranslated region of E2 messenger RNA from a papillomavirus, that inhibits the function of said messenger RNA when hybridised therewith.

2. An oligonucleotide or oligonucleotide analogue comprising from 8 to 50 bases, hybridisable with an E2 messenger RNA from a papillomavirus, that inhibits the function of said messenger RNA when hybridised therewith, for use as a pharmaceutical in a method of treatment or prophylaxis.

3. The use of an oligonucleotide or oligonucleotide analogue as defined in claim 2 in the manufacture of a medicament for the treatment of a papillomavirus infection in an animal.

4. A method for detecting the presence or absence of papillomavirus in a sample, comprising contacting the sample with an oligonucleotide of sequence: any one of SEQ ID NOs: 1 to 6,
or an analogue thereof, and detecting any hybridisation.

5. A kit for the detection of the presence or absence of papillomavirus in a sample, comprising an oligonucleotide selected from sequences: any one of SEQ ID NOs: 1 to 6,
or an analogue thereof, and means for detecting any hybridisation.

## Patentansprüche

1. Oligonucleotid oder Oligonudeotidanalogon, das 8 bis 50 Basen umfaßt und mit einem Transaktivator-Abschnitt, einem Transrepressor-Abschnitt, einer cap-Region oder einer 5'-nicht-translatierten Region der E2 Messenger-RNA aus einem Papillomavirus hybridisierbar ist, das nach der Hybridisierung damit die Funktion der genannten Messenger-RNA hemmt (inhibiert).

2. Oligonucleotid oder Oligonucleotidanalogon, das 8 bis 50 Basen umfaßt und mit einer E2 Messenger-RNA aus einem Papillomavirus hybridisierbar ist, das nach der Hybridisierung damit die Funktion der genannten Messenger-RNA hemmt (inhibiert), für die Verwendung als Pharmazeutikum in einem Verfahren zur Behandlung oder Prophylaxe.

3. Verwendung eines Oligonucleotids oder Oligonucleotidanalogons nach Anspruch 2 bei der Herstellung eines Arzneimittels für die Behandlung einer Papillomavirus-Infektion eines Tieres.

4. Verfahren zum Nachweis der Anwesenheit oder Abwesenheit des Papillomavirus in einer Probe, das umfaßt das Inkontaktbringen der Probe mit einem Oligonucleotid mit der Sequenz: irgendeiner der SEQ ID Nr. 1 bis 6 '
oder einem Analogon davon und den Nachweis einer Hybridisierung.

5. Kit für den Nachweis der Anwesenheit oder Abwesenheit des Papillomavirus in einer Probe, der umfaßt ein Oligonucleotid, ausgewählt aus den Sequenzen: und irgendeiner der SEQ ID Nr. 1 bis 6
oder eines Analogons davon und ein Mittel zum Nachweis einer Hybridisierung.

## Revendications

1. Oligonucléotide ou analogue d'oligonucléotide comprenant de 8 à 50 bases, susceptible d'être hybridé à un fragment transactivateur, à un fragment transrépresseur, à la région de la coiffe ou à la région 5' non traduite de l'ARNm de E2 issu d'un virus du papillome, qui inhibe l'activité dudit ARNm lorsqu'il s'hybride à ce dernier.

2. Oligonucléotide ou analogue d'oligonucléotide comprenant de 8 à 50 bases, susceptible d'être hybridé à un ARNm de E2 issu d'un virus du papillome, qui inhibe l'activité dudit ARNm lorsqu'il s'hybride à ce demier, pour utilisation à titre de médicament dans une méthode de traitement ou en prophylaxie.

3. Utilisation d'un oligonucléotide ou analogue d'oligonucléotide selon la revendication 2 dans la préparation d'un médicament pour le traitement d'un animal infecté par un virus du papillome.

4. Procédé de détermination de la présence ou de l'absence de virus du papillome dans un échantillon, comprenant les étapes consistant à mettre en contact l'échantillon avec un oligonucléotide de séquence : l'une quelconque des SEQ ID Nos : 1 à 6,
ou un de ses analogues et à détecter toute hybridation.

5. Trousse de détermination de la présence ou de l'absence de virus du papillome dans un échantillon, comprenant un oligonucléotide choisi parmi les séquences suivantes : l'une quelconque des SEQ ID Nos : 1 à 6,
ou un de leurs analogues, et des moyens pour détecter toute hybridation.
